# EUROPEAN PATENT APPLICATION

(11) **EP 2 436 332 A1**
(43) Date of publication of application: **04.04.2012**
(21) Application number: 10012541.8
(22) Date of filing: 30.09.2010
(51) Int. Cl.: A61B 18/20

(54) **Light-based skin treatment device**

(71) Applicant: Braun GmbH, 61476 Kronberg/Taunus (DE)
(72) Inventor: Wurl, Andreas, 61476 Kronberg/Taunus (DE); Mandre, Shyam K., 64850 Schaafheim (DE); Kruch, Torben, 65835 Liederbach (DE); Beerwerth, Frank, 65558 Kaltenholzhausen (DE)

(57) **Abstract**

The present invention is concerned with a light-based skin treatment management device that comprises a light source unit (200) comprising a light source (210) for emission of light being effective for a skin treatment, an optical unit (100) having a light entrance window (110) arranged at an light entrance side (111), a semitransparent light output window (120) arranged at a light output side (121) for applying light to a skin area, and at least a side wall (130) arranged to reflect the light, and the light source unit (200) is arranged such that light emitted by the light source (210) enters the optical unit (100) through the light entrance window (110). The optical unit (100) serves to generate a light output beam of superimposed partial beams that each relate to a different (virtual) light source, so that the eye-safety of the light output beam is improved.

## Description

### FIELD OF THE INVENTION

The present invention is concerned with light-based skin treatment devices comprising a light source unit for emission of light being effective for a skin treatment and in particular with such a light-based skin treatment device that further comprises an optical unit.

### BACKGROUND OF THE INVENTION

Light-based skin treatment devices are widely known, e.g. for hair removal, skin rejuvenation, acne treatment etc. In particular, professional hair removal devices or hair removal devices suitable for use by consumers at home are known.

For light-based skin treatment devices it is of particular interest that they can be safely used at home by a consumer. E.g. in US 7,452,356 a dermatologic treatment apparatus is disclosed which includes one or more housings with at least one housing configured for manipulation in a dermatologic treatment procedure, a light source, and an electrical circuit. The circuit energizes the light source to produce output light pulses. A light path includes an aperture through which eye-safe light pulses are propagated having properties sufficient for providing efficacious treatment. An optical diffuser is disposed along the light path to reduce the integrated radiance to an eye-safe level.

### SUMMARY OF THE INVENTION

It is hence a desire to provide a light-based skin treatment device that is specifically suitable for home use.

Such a light-based skin treatment device is given in accordance with claim 1. Further embodiments are given by the dependent claims.

A light-based skin treatment device as proposed has a light source unit and an optical unit. The light source unit comprises a light source that in operation emits light that is effective for a skin treatment (i.e. the wavelength and the intensity of the light source are such that the output beam applied onto the skin will lead to the desired skin treatment effect). The optical unit comprises a light entrance window arranged at a light entrance side, a semi-transparent light output window arranged at a light output side and at least a side wall. Depending on the geometry of the optical unit, the various sides may merge into each other without a geometrically given edge separating the sides (e.g. the optical unit may be realized as a sphere). In some embodiments, the light entrance window may cover the light entrance side completely and/or the semi-transparent light output window may cover the light output side completely. The side wall (or side walls) is (are) arranged for reflecting the light that has entered the optical unit. Reflection may be achieved via a reflecting layer or through total internal reflection (TIR). The light output window is arranged to apply light onto a skin area. Application of light may happen via direct contact of the light output window with the skin or via a further light path. Further, the light source is arranged so that light that is emitted by the light source during operation enters the optical unit through the light entrance window. The light (or at least part of it) that enters the optical unit may directly impinge onto the semi-transparent light output window or the light may need to be reflected at least once before it impinges onto the semi-transparent light output window. The semi-transparent light output window transmits a part of the light that impinges onto it and reflects the remainder back into the optical unit.

The light beam that is emitted by the light source and that enters the optical unit will only partially leave the optical unit through the semi-transparent window. The not transmitted beam is reflected by the semi-transparent light output window back into the optical unit. As the side walls are arranged for reflecting the light (and further those parts of the light entrance side and of the light output side that are not covered by the light entrance window and the semi-transparent light output window, respectively, are also arranged for reflecting the light inside the optical unit), the reflected beam will ultimately impinge onto the semi-transparent light output window a second and further times until all light has left the optical unit or is absorbed in the optical unit. Some light will eventually leave the optical unit via the light entrance window. The design of the optical unit may be chosen to make this fraction of light as low as possible, e.g. by choosing a light entrance window that is geometrically as small as possible.

In an embodiment, the semi-transparent light output window has a transmission rate of 0.1 % - 30% at the wavelength or the wavelength range of the light emitted by the light source at zero degrees incident angle, where the incident angle is defined with respect to the surface normal of the semi-transparent light output window so that zero degrees incident angle means a light beam that perpendicularly impinges onto the semi-transparent light output window. For example, the transmission rate of the semi-transparent light output window is 3% - 4% at the wavelength or the wavelength range of the light emitted by the light source at zero degrees incident angle.

In another embodiment, the divergence of the light beam that is emitted by the light source unit has a value of 60 degrees - 70 degrees (FWHM) in at least one direction. In particular, the divergence values may be chosen such that at a given transmittance of the semi-transparent light output window the optical loss induced by the optical unit is kept as low as possible.

In an embodiment, the light source unit comprises a diverging unit, which is used to make the light beam emitted by the light source divergent or even more divergent.

In a further embodiment, the optical unit comprises a block of bulk material being essentially transparent at the wavelength or wavelength range of the light emitted by the light source. The light entrance side, the semi-transparent light output side, and the side wall are provided at the block of bulk material. For example, the bulk material has a coefficient of internal transmission of at least 0.997 for a material thickness of 25 mm at the wavelength or the wavelength range of the light emitted by the light source. This assures that the internal light losses in the optical unit due to absorption in the bulk material are at an acceptable level.

In an alternative embodiment, the optical unit comprises a hollow casing at which the light entrance side, the semi-transparent light output side, and the side wall are arranged. For example, the hollow casing is coated with a reflective coating on the inner side of the hollow casing except for the light entrance window and the semi-transparent light output window.

In another embodiment, the light entrance side has a reflective coating on its inner side (i.e. the side opposite of the side onto which the light will initially impinge when emitted by the light source) except for the light entrance window. The reflective coating is arranged to reflect light that has entered the optical unit back into the optical unit so that it can leave the optical unit only via the light entrance window or through the semi-transparent light output window. For example, the light output side has a reflective coating except for the semi-transparent light output window.

In an even further embodiment, the side wall has a reflective coating that has a reflection coefficient of at least 0.99 at the wavelength or the wavelength range of the light emitted by the light source at zero degrees incident angle. For example, the reflection coefficient may be chosen to be at least 0.996. This allows keeping light losses via transmission at an acceptable level.

In yet another embodiment, the distance between the light entrance window and the semi-transparent light output window lies in a range of about 10 mm and 100 mm.

In an embodiment, the light source unit comprises a light coupling unit for optically coupling the light emitted by the light source to the light entrance window.

In another embodiment, the light source is directly attached to the light entrance window.

### BRIEF DESCRIPTION OF THE DRAWINGS

The invention will be further elucidated by detailed explanation of exemplary embodiments and by reference to figures. In the figures
- Fig. 1: is a schematic depiction of an exemplary light-based skin treatment device as proposed with a skin contact part shown in a detached state;
- Fig. 2A: is a schematic depiction of a perspective onto the light entrance side of an optical unit as may be utilized in a light-based skin treatment device;
- Fig. 2B: is a schematic depiction of a perspective onto the light output side of an optical unit as may be utilized in a light-based skin treatment device;
- Fig. 3: is schematic cross-sectional view through an optical unit and a light source unit of a first embodiment;
- Fig. 4: is schematic cross-sectional view through an optical unit and a light source unit of a second embodiment; and
- Fig. 5: is schematic cross-sectional view through an optical unit and a light source unit of a third embodiment.

### DETAILED DESCRIPTION OF THE INVENTION

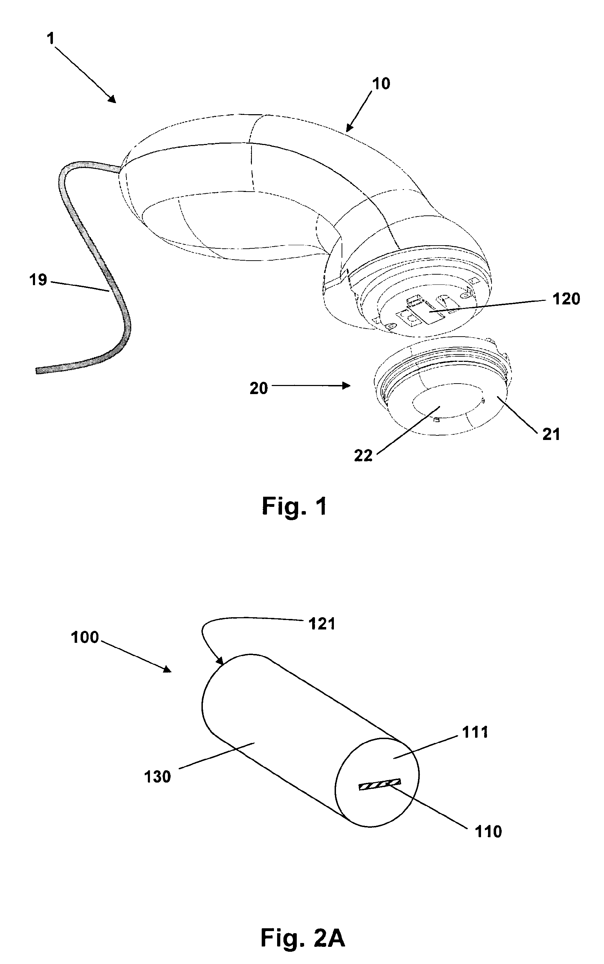

Fig. 1 shows a schematic depiction of an exemplary embodiment of a light-based skin treatment device 1 as proposed. The light-based skin treatment device 1 has a hand-piece part 10 that is intended to be held by a user's hand. In the shown embodiment, the light-based skin treatment device 1 is energized via a cord 19 that can be connected to mains voltage (e.g. via a power supply), but the light-based skin treatment device 1 could additionally or alternatively also be energized by a battery or an accumulator arranged in the device. The light-based skin treatment device 1 has a skin contact part 20 that is shown in a detached state. The skin contact part 20 has a skin contact pillow for smooth and gentle contact with the skin and a light transmission window 22. At the skin side of the hand-piece part 10 a light output window 120 of an optical unit is arranged. In the shown embodiment, the light that exits the light output window 120 during operation will further traverse the light transmission window 22 of the skin contact part 20 before the light impinges onto the skin. In a different embodiment, the light output window 120 of the light-based skin treatment device 1 is arranged to directly contact the skin in regular operation. In even another embodiment, a cooling liquid-streamed or air-streamed channel may be present between the light output window 120 and the transmission window 22 to carry away waste heat. Hence, the light output window 120 may be in direct contact with the skin or may apply light onto the skin via a further medium (or via further media) such as an air gap or a further light window (or a combination of an air gap and a further light window). The light-based skin treatment device 1 comprises a light source unit that itself comprises a light source such as a laser, a laser diode, a laser diode bar, a LED or an array of LEDs, or a VCSEL (Vertical-Cavity Surface-Emitting Laser) or an array of VCSEL. The light source is arranged to controllably emit light at a given wavelength or within a given wavelength range (i.e. a control unit is coupled to the light source). In case the light-based skin treatment device 1 is realized as a hair growth management device, the light source may be realized as a laser diode bar that emits light at about 810 nanometers (nm), as melanin present in hair follicles strongly absorbs at this wavelength. For different applications such as skin rejuvenation, scar treatment, acne treatment etc., a different wavelength may be chosen that is adapted to the absorbing substance so that a skin treatment is enabled.

A light-based skin treatment device is specifically suitable for utilization by an end consumer if the light that exits the device cannot damage the retina of the eye. In particular, such a device could fulfill the requirements of a Class I laser device as is given by the standard IEC 60825-1. In essence, this standard defines the maximum intensity (in dependence on wavelength and time of exposure) of light that can be emitted by a point source that is focused onto the retina so that the laser source is considered as eye-safe. The light intensity required to effect e.g. at least a temporal hair re-growth inhibition lies beyond this threshold. In order to overcome this issue, it is known to use a diffuser to increase the divergence and destroy the spatial coherence of the laser beam. The eye can then not focus anymore onto the point-like light source but the diffuser itself becomes an extended light source. This was in general described e.g. by D. Sliney and M. Wolbarsht in "Safety with lasers and other optical sources", 1980, Plenum Press, New York (e.g. on pages 477-480) and was specifically discussed for dermatological treatment apparatuses in e.g. US 7,452,356 and in US 7,184,614. In the following, it is proposed to utilize an optical unit having a semi-transparent light output window and reflecting walls so that a light beam emitted by a light source is converted into an output beam that is a superposition of a multiplicity of partial output beams that each relate to a different (virtual) light source. While this is not a diffuser, it also reduces the intensity of light imaged onto the retina as will be explained in more detail below. This proposed optical unit may be used in conjunction with a beam diverging unit to further reduce the light intensity that is focused onto the retina. In total, eye safety of a light-based skin treatment device can be achieved without the need of a diffuser.

Fig. 2A is a schematic depiction of a perspective view onto a light entrance side 111 of an optical unit 100 that may be arranged in a light-based skin treatment device 1 as shown in Fig. 1. The optical unit 100 may be made from a highly transparent bulk material, e.g. a glass material such as Schott F2, Schott LF5, or Schott Lithosol-Q. E.g., Schott F2 has a transmittance value of about 0.998 at a material length of 25 mm and at a wavelength of 810 nm. Hence, absorption losses of light emitted from a laser diode bar at 810 nm are low in such a material. The light entrance side 111 of the optical unit 100 is coated with a highly reflective material, which may be realized as a thin reflective layer. The thin reflective layer may be realized as a metal layer, even though the light losses in such a metal layer would be high. As it is a desire to apply as much light onto the skin and to minimize losses, the reflective layer may for example be realized as a dichroic mirror (a stack of dielectric thin layers) which is optimized for the wavelength or wavelength range of light that is emitted by the light source. E.g. a dichroic mirror having a reflectivity of about 0.99 or higher at a wavelength of 810 nm could be chosen. For example, a dichroic mirror having a reflectivity of about 0.996 at a wavelength of 810 nm could be chosen. The transmissivitiy of a reflective layer such as a dichroic mirror changes with the incidence angle but is usually almost constant at low incidence angles. E.g. LASEROPTIK GmbH, D-30826 Garbsen, Germany is a provider of customized high reflective coatings such as dichroic mirrors.

Further, a light entrance window 110 is arranged at the light entrance side. The light entrance window may be realized as an uncoated part of the light entrance side 111. In order to minimize losses of light inside the optical unit 100, the light entrance window should be realized as small as possible as reflected light that impinges onto the inner side of the light entrance side 111 will leave the optical unit 100 through the uncoated light entrance window 110. E.g. in case a single laser diode bar is utilized as light source, the light entrance window 110 could have an area of 10 millimeters (mm) times 0.2 mm = 2 mm², while the area of the light entrance side 111 could be about 2 cm². The light source may be directly coupled to the light entrance window, e.g. using an index matched optical glue.

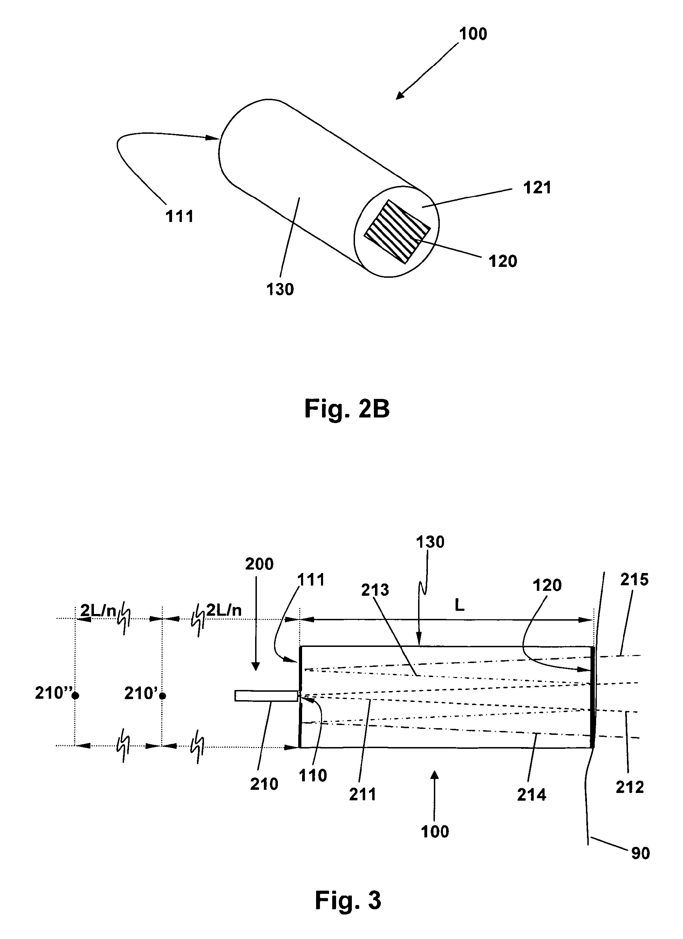

In the shown embodiment, the optical unit 100 is a glass cylinder (e.g. made from Schott LF5) that has a side wall 130 that in the shown embodiment is uncoated. As the light entrance side 111 and the light exit side 121 (see Figs. 2B and Fig. 3) are realized as being flat and parallel to each other and the side wall 130 is further realized with a 90 degrees angle with respect to the light entrance side 111 and the light exit side 121, light entering the optical unit 100 cannot leave the optical unit 100 through the side wall 130 due to the principle of total internal reflection (TIR). In case, a differently shaped optical unit 100 that deviates from these principles (e.g. non-parallel light entrance side 111 and light exit side 121) would be chosen or a different geometrical arrangement of light entrance window 111 and light exit window (see Figs. 2B and 3) would be chosen, a coating of the side wall (e.g. realized again as a dichroic mirror) will be required to keep light exit losses low. Generally, a geometrical design as discussed that does not require a coated side wall 130 is optimized for low light loss at the side wall 130.

Fig. 2B is a schematic depiction of a perspective view onto the light exit side 121 of the optical unit 100 as shown in Fig. 2A. A semi-transparent light output window 120 is arranged on the light exit side 121. In the present case, the optical unit 100 has a cylindrical shape and the light entrance side 111 and the light exit side 121 are both circular surfaces. In the present embodiment, the semi-transparent light output window 120 is realized as a rectangular semi-transparent window within the circular light exit side 121, which light exit side 121 is elsewhere coated with a dichroic mirror. The semi-transparent coating may also be realized as a stack of dielectric layers such that the properties of the semi-transparent window can be adapted to the wavelength of the light source or the wavelength range that is emitted by the light source. The transmission rate may be designed to lie in the range of T = 0.1% - 30% (again, LASEROPTIK GmbH is a provider of such customized partial transmission windows).

Instead of being made from a bulk material, the optical unit 100 can also be made from a hollow (e.g. air-filled) cylinder, where the light entrance window is realized as a small opening in the light entrance side of the hollow cylinder. In such a realization, the side wall of the hollow cylinder should be coated with a reflecting layer. The reflecting layers are for example arranged on the inner sides of the walls (i.e. the light entrance side, the light exit side, and the side wall) of the hollow cylinder to avoid any losses in the wall material.

Generally, even though the absorption of light in air is very low, the overall losses in an optical unit realized as a hollow cylinder are higher than the losses in a comparable optical unit made from bulk material as the reflective layers in a hollow cylinder are usually metallic layers (e.g. a silver (Ag) layer). E.g. an optical unit made from bulk material can be designed to have an overall light loss of less than 20% while a comparable optical unit comprising a hollow cavity with a reflective Ag layer and a semi-transparent light exit window having a transmission rate of 1.9% may easily have a light loss of about 50%.

Fig. 3 is a schematic depiction of a cross sectional cut through the centre of an optical unit 100 together with a light source unit 200 that comprises a light source 210. The optical unit 100 may be shaped as a cylinder as was shown in Figs. 2A and 2B or as a rectangular prism. In the latter case, the semi-transparent light output window 120 may cover the full surface area of the rectangular light exit side 121. The optical unit 100 hence comprises a rectangular prism 101 made from bulk material such as Schott Lithosol-Q, a light entrance window 110 realized as an uncoated section or alternatively an anti-reflection coated section in the otherwise high-reflection-coated light entrance side 111, where the reflective coating is a dichroic mirror. The side walls 130 are uncoated as the shown embodiment makes use of total internal reflection (TIR), so that the losses due to a coating of the side walls 130 are avoided. A light source unit 200 that comprises a light source (here: a laser diode bar emitting at 810 nm) is arranged such that in operation the emitted light enters the optical unit 100 through the light entrance window 110 in the form of a diverging fan 211. Depending on the transmission rate T (e.g. 1% - 3%; as will be discussed further below, a high transmission rate needs to be compensated by a high beam divergence to achieve an eye-safe treatment beam) of the semi-transparent light output window 120 realized as a stack of dielectric layers forming a semi-transparent window, a primary treatment fan 212 of light of a certain radiancy exits the optical unit 100 and impinges onto the skin 90, where it scatters and will partially be absorbed by melanin in the hair follicles to effectuate a (partial) coagulation that ultimately leads to an at least temporal hair growth inhibition. The larger part of the diverging light fan 211 will be reflected by the semi-transparent light output window 120 and forms a reflected fan 213. The reflected fan 213 will mostly be reflected again by the dichroic mirror forming the light entrance side 111 to result in a twice-reflected fan 214. Depending on the geometry and the fan divergence, the reflected fan 213 and/or the twice-reflected fan 214 may also be TIR-reflected at the side wall(s) 130. A part of the reflected fan 213 will exit the optical unit 100 through the uncoated light entrance window 110. Depending on the transmission rate T, a part of the twice-reflected fan 214 will exit the optical unit 100 as secondary treatment fan 215 via the semi-transparent light output window 120 and will impinge onto the skin 90. The combined fluence of the successively emitted treatment fans will lead to the above mentioned hair growth inhibition effect. The reflection and transmission of the fans will continue until all light has exited the optical unit 100 or is absorbed by the optical unit 100. By a design as was described, a total light usage of up to 90% can be achieved at a wavelength of 810 nm.

The transmission rate T of the semi-transparent light output window 120 should be chosen such that the light source 210 seen by a focusing eye as origin of the primary treatment fan 212 should not have a radiance above a threshold that would be unsafe for the eye (such radiance values are e.g. given by the standard IEC 60825-1). The secondary treatment fan 215 (and the further n-ary treatment fans that exit the optical unit 100 through continued reflection and transmission of the original light fan 211) can be seen by the eye as a virtual light source 210' at a distance 2L/n (n: refractive index of material of optical unit) from the real light source 210 as the light has travelled twice the length L of the optical unit 100. A further virtual light source 210" will be seen at a distance 4L/n from the real light source 210 and so on. When the eye has focused onto an object at a certain distance it will see farther away lying virtual light sources (or closer lying light sources) only as blurred light sources and not as essentially point sources. Even though the discussed system does not work as an optical diffuser that destroys coherence, the proposed system makes use of the focusing abilities of the human eye. Hence, the light intensity of all virtual light sources imaged onto the retina of the focusing eye can be adapted to lie within the upper limits defined by above mentioned standards.

It was found that the length L of the optical unit may be chosen to lie in a range of about 10 mm to about 40 mm for a given light loss below about 30%. Nevertheless, if a higher light loss would be allowable, an even longer optical unit can be used, e.g. having a length of L = 100 mm, which would improve the eye-safety of the light-based skin treatment device. As a light-based skin treatment device must deliver a light intensity that effectuates a skin treatment effect (e.g. hair re-growth inhibition by at least partially coagulating the hair follicle), the tolerable length of the optical unit (and hence the tolerable light loss) ultimately depends on the light emission power of the light source and the waste heat dissipation capability of the device.

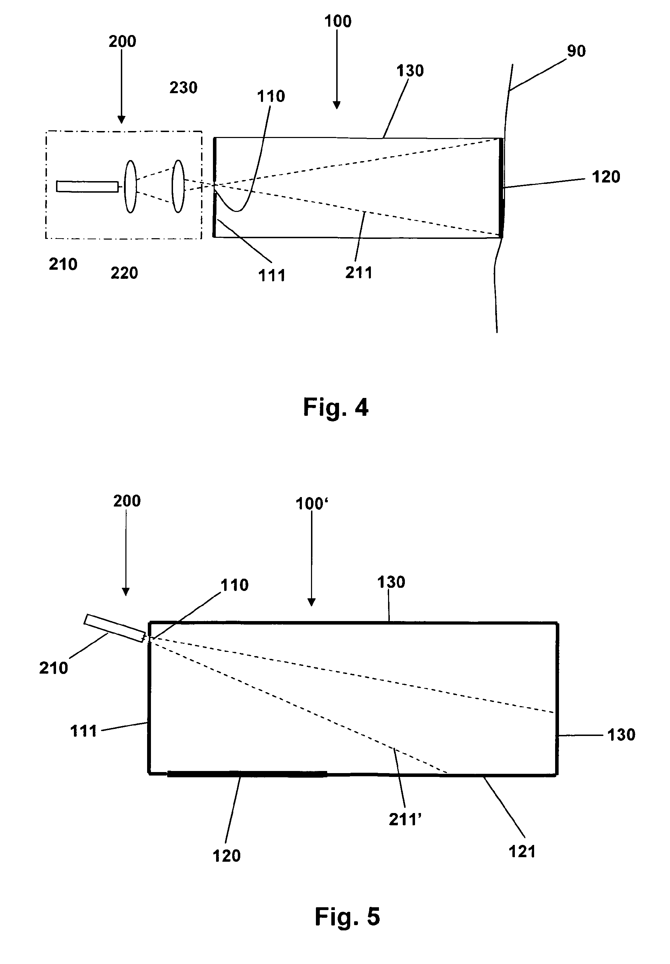

In Fig. 4, a schematic depiction of a cross-sectional cut through a light source unit 200 and an optical unit 100 is shown. The optical unit 100 is identical to the optical unit as discussed with reference to Fig. 3. The optical unit 200 comprises a light source 210 (here: a laser diode bar), a beam diverging unit 220, and a light coupling unit 230. The beam diverging unit 220 is arranged to make the incident light beam divergent (or in case that an already divergent light beam impinges onto the beam diverging unit 220: to make the light beam even more divergent than it is before the beam diverging unit 220). A light beam that is emitted by a laser diode bar has a typical divergence in axial direction of about 6 degrees (slow axis divergence). The beam diverging unit 220 may increase this value to a divergence angle of up to about 60 degrees - 70 degrees (at larger divergence angles the transmission rate of the dichroic mirrors may increase to intolerable values due to the incidence angle dependence of the transmission rate or such dichroic mirrors will become to expensive). The divergence in the fast axis of a laser diode bar typically lies above the divergence in the slow axis; a divergence unit may nevertheless be used to also increase the divergence in the fast axis. The divergence of the emitted light beam has a direct influence on the intensity of the light beam that is imaged onto the retina. Hence, an increase in the divergence of the emitted light beam helps in achieving the required threshold of a Class I laser device. A light coupling unit 230 is used to couple the divergent beam onto the light entrance window 110 to allow that the light entrance window 110 can be designed small.

Fig. 5 is schematic depiction of a cross sectional view through a different embodiment of an optical unit 100' and a light source unit 200 as proposed for a light-based skin-treatment device. In the shown embodiment, the optical unit 100' is realized as a hollow cubical casing (any other geometrical form may likewise be possible; instead of being hollow, the optical unit may made from a bulk material such as Schott Lithosol-Q). The side walls 130 of the hollow cubic casing have a reflecting coating provided on the inner side of the side walls 130. The reflective coating may e.g. be realized as a metal layer (the advantage of a metal layer is the essentially low incident angle dependence of its reflectivity, whereas the disadvantage of a metal layer is its relatively high absorption value). The light entrance side 111 is provided on a small side of the casing and the light exit side 121 is provided at a wide side of the cubic casing. A light entrance window 110 is provided in the light entrance side 111 and a light source unit 200 comprising a light source 210 such as a laser diode bar is arranged such that the beam of light 211' emitted by the light source 210 enters the hollow cubic casing through the light entrance window 110. A semi-transparent light output window 120 realized as a semi-transparent mirror is provided on the light exit side 121 such that the primary light fan 121' will not exit the hollow cubic casing through the semi-transparent light output window 120. As was explained with respect to Fig. 3, the primary fan 211' will be repeatedly reflected by the walls of the cubic casing and parts of a reflected beam that impinges onto the semi-transparent light output window 120 will exit the cubic casing depending on the transparency of this semi-transparent light exit window. Surface inhomogeneities may be arranged into the reflecting walls such that the beam is spatially widened or may even loose its spatial coherence. This means that the virtual light sources seen by a user's eye are blurred as such and the required threshold values for a Class I laser device can be achieved.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. Light-based skin treatment device comprising:
a light source unit (200) comprising a light source (210) for emission of light being effective for a skin treatment;
an optical unit (100) having a light entrance window (110) arranged at an light entrance side (111), a semi-transparent light output window (120) arranged at a light output side (121) for applying light to a skin area, and at least a side wall (130) arranged to reflect the light; and
wherein the light source unit (200) is arranged such that light emitted by the light source (210) enters the optical unit (100) through the light entrance window (110).

2. Light-based skin treatment device according to claim 1, wherein the semi-transparent light output window (120) has a transmission rate of about 0.1% - 30% at the wavelength or over the wavelength range of the emitted light at zero degrees incident angle.

3. Light-based skin treatment device according to claim 2, wherein the semi-transparent light output window (120) has a transmission rate of about 3% - 4% at the wavelength or over the wavelength range of the emitted light at zero degrees incident angle.

4. Light-based skin treatment device according to one of claims 1 to 3, wherein the divergence of the light emitted by the light source unit (200) has a value of about 60 degrees to 70 degrees in at least a direction.

5. Light-based skin treatment device according to one of claims 1 to 4, wherein the light source unit (200) comprises a diverging unit (220).

6. Light-based skin treatment device according to one of claims 1 to 5, wherein the optical unit (100) comprises a block of bulk material at which the light entrance side (111), the light output side (121), and the side wall (130) are provided.

7. Light-based skin treatment device according to claim 6, wherein the bulk material has a coefficient of internal transmission of at least about 0.997 for a material thickness of 25 mm at the wavelength or over the wavelength range of the emitted light.

8. Light-based skin treatment device according to one of claims 1 to 7, wherein the optical unit (100) comprises a hollow casing at which the light entrance side (111), the light output side (121), and the side wall (130) are arranged.

9. Light-based skin treatment management device according to one of claims 1 to 8, wherein the light entrance side (111) has a reflective coating except for the light entrance window (110).

10. Light-based skin treatment device according to one of claims 1 to 9, wherein at least the side wall (130) has a reflective coating that has a reflection coefficient of at least 0.99 at the wavelength or over the wavelength range of the emitted light at an incidence angle of zero degrees.

11. Light-based skin treatment device according to one of claims 1 to 10, wherein the distance between the light entrance window (110) and the semi-transparent light output window (120) lies in a range of about 10 mm to about 100 mm.

12. Light-based skin treatment device according to one of claims 1 to 11, wherein the light source unit (200) comprises a light coupling unit (230) for optically coupling the light source (210) to the light entrance window (110).

13. Light-based skin treatment device according to one of claims 1 to 12, wherein the light source (210) is directly attached to the light entrance window (110).
